# EUROPÄISCHE PATENTSCHRIFT

(11) **EP 2 729 801 B1**
(45) Veröffentlichungstag und Bekanntmachung des Hinweises auf die Patenterteilung: **30.08.2017**
(21) Anmeldenummer: 12787853.6
(22) Anmeldetag: 06.07.2012
(51) Int. Cl.: G01N 33/497

(54) **VORRICHTUNG UND VERFAHREN ZUR ANALYSE VON FLÜCHTIGEN ORGANISCHEN VERBINDUNGEN IN GASFÖRMIGEN PROBEN**
DEVICE AND METHOD FOR ANALYZING VOLATILE ORGANIC COMPOUNDS IN GASEOUS SAMPLES
DISPOSITIF ET PROCÉDÉ D'ANALYSE DE COMPOSÉS ORGANIQUES VOLATILS DANS DES ÉCHANTILLONS GAZEUX

(30) Priorität: 06.07.2011 DE 102011106717
(43) Veröffentlichungstag der Anmeldung: 14.05.2014
(73) Patentinhaber: Becher Consult MD, 16321 Bernau (DE)
(72) Erfinder: BECHER, Gunther, 16321 Bernau (DE); PURKHART, Roman, 12459 Berlin (DE); VIERTEL, Beate, 09419 Thum (DE); GRAUPNER, Rolf, 09353 Oberlungwitz (DE)
(74) Vertreter: Baumbach, Friedrich
(86) Internationale Anmeldenummer: PCT/IB2012/001924
(87) Internationale Veröffentlichungsnummer: WO 2013/011381

(56) Entgegenhaltungen:
- DE-A1-102007 033 906
- US-A1- 2007 003 996
- ROMAN PURKHART ET AL: "Chronic intestinal Mycobacteria infection: discrimination via VOC analysis in exhaled breath and headspace of feces using differential ion mobility spectrometry", JOURNAL OF BREATH RESEARCH, Bd. 5, Nr. 2, 1. Juni 2011 (2011-06-01), Seite 027103, XP55048294, ISSN: 1752-7155, DOI: 10.1088/1752-7155/5/2/027103
- A. KIKOWATZ ET AL: "Differential ion mobility spectroscopy: non-invasive real-time diagnostics and therapy control in metabolic diseases", EUROPEAN JOURNAL OF MEDICAL RESEARCH, Bd. 14, Nr. Suppl. 4, 7. Dezember 2009 (2009-12-07), Seiten 121-125, XP55048299, ISSN: 0949-2321, DOI: 10.1186/2047-783X-14-S4-121
- ANDREW G. ANDERSON ET AL: "<title>DMS-IMS2, GC-DMS, DMS-MS: DMS hybrid devices combining orthogonal principles of separation for challenging applications</title>", PROCEEDINGS OF SPIE, Bd. 6975, 3. April 2008 (2008-04-03), Seite 69540H, XP55048326, ISSN: 0277-786X, DOI: 10.1117/12.782429

## Beschreibung

Die Erfindung betrifft Vorrichtungen und Verfahren zur Analyse von flüchtigen organischen Verbindungen in gasförmigen Proben und Kategorisierung der gasförmigen Proben. Dabei wird eine vergleichende Mustererkennung und Bewertung in verschiedenen mehrdimensional aufgezeichneten Spektren, beispielsweise aus Gaschromatographie oder lonenbeweglichkeitsspektrometrie durchgeführt. Vorwiegend dient das Verfahren zur Analyse von flüchtigen organischen Substanzen (VOC's) in Atemluft und auch anderen direkt oder indirekt gewonnenen biologischen Proben mit dem Ziel, die erzeugten Spektrogramme in Kategorien einzuordnen und die Zuordnung weiterer Spektrogramme zu ermöglichen, ohne dass die einzelnen Substanzen aufwendig identifiziert werden müssen. Anwendungsgebiete sind die medizinische Diagnostik, die Biotechnologie, Technik sowie die analytische Chemie, aber auch der Gesundheitsschutz und die medizinische Vorsorge auf der Basis von Emissions- und Arbeitsplatzmessungen, die sowohl stationär als auch personenbezogen erfolgen können.

### Beschreibung

Es ist allgemein bekannt, dass in der Ausatemluft von Tier und Mensch mehrere hundert verschiedene chemische Substanzen nachgewiesen werden können. Die Literatur lässt sich zurückverfolgen bis GALEN, der schon festgestellt hatte, dass aus dem Geruch der Ausatemluft ("Phoeter ex orae") Rückschlüsse auf Krankheiten ziehen lassen. Der seit damals bekannte Geruch bei Diabetes beruht vermutlich auf Ketonkörpern in der Ausatemluft.
Linus Pauling (Quantitative Analysis of Urine Vapor and Breath by Gas-Liquid. Partition Chromatography Proc. Nat. Acad. Sci. USA, Vol. 68, No. 10, pp. 2374-2376, October 1971) hat 1971 Messungen dazu mit Hilfe der Gaschromatographie beschrieben. Dabei wurde Atemluft sowohl direkt im Gaschromatographen gemessen, als auch aus dem verdampften Kondensat der Ausatemluft, das zuvor in einer Kühlfalle gesammelt wurde.

Messungen werden gegenwärtig typischerweise mit spektrometrischen Verfahren durchgeführt, z.B. mit Ionenbeweglichkeitsspektrometrie (IMS) oder Differentieller lonen-Mobilitäts-Spektrometrie (DMS). Möglich sind auch Kombination dieser Verfahren mit Säulen (Gaschromatographiesäulen) zur Vortrennung von Proben und der parallelen und seriellen Kopplung verschiedener Sensoren (WO 2007/05488).

Gemeinsames Problem aller Arbeiten zur Messung von chemischen Substanzen in der Atemluft bleibt die Standardisierung der Probennahme und die Bewertung der in den Spektrogrammen gefundenen Peaks, zumal die Identifizierung der Peaks zur Zuordnung zu bestimmten Substanzen weitgehend noch nicht gelöst ist. Das ist einsehbar, wenn man bedenkt, dass in einem Atemzug Einzelsubstanzen in Konzentrationen vom ppb (Parts per Billion) oder sogar ppt (Parts per Trillion) zu finden sind.
Man behilft sich verschiedener Methoden der Probengewinnung (z.B., M. Phillips über ein beheiztes Atemrohr) oder der weitgehenden Standardisierung der Probe über "Ausschluss aller Fremdsubstanzen" (J. Baumbach "Klassifizierungs- und Unterscheidungsmodelle für Lungenerkrankungen mittels lonenbeweglichkeitsspektrometrie mit BreathDiscovery, Breath Discovery based on Ion Mobility Spectrometry and Classification and Differentiation Models for Lung Diseases." Biomed Tech 2010; 55 (Suppl. 1) 2010, DOI 10.1515/BMT.2010.537), was über Nahrungskarenz, Verzicht auf Zahnpaste, Kaugummis und zusätzlich extensive Mundspülungen hinarbeitet. Weiter versuchen viele Autoren, gemessene Spektren der Umgebungsluft von den Spektren der Ausatemluft abzuziehen. Dieses Verfahren führt zu negativen Peaks in der Ausatemluft, was darauf hinweist, dass Substanzen der Umgebungsluft durch die Atmungsorgane resorbiert werden. Das ist kein überraschendes Ergebnis, da ja bekannt ist, dass die Lunge die physiologische Aufgabe hat, z.B. Sauerstoff aus der Luft aufzunehmen, aber auch andere Stoffe abzugeben. Durch das Abzeihen der Messungen der Umgebungsluft von der Ausatemluft wird das Ergebnis aber möglicherweise verfälscht, jedenfalls immer dann, wenn bestimmte Marker sowohl in der Umgebung als auch in der Ausatemluft auftreten!
Eine weitere Möglichkeit ist die Präklassifizierung von Daten bzw. Datensets, wo bestimmte Peaks oder Spektrogramme bestimmten Krankheiten zugeordnet werden (Baumbach et al., 2007, J Integr Bioinf 4, 75). Dafür ist jedoch die sichere Erkennung der Krankheit und entsprechende Zuordnung des oder der Spektrogramme zu einer Krankheit oder einem Zustand notwendig. Bekannt ist auch die Vordefinition zur Detektion von bekannten Substanzen, die man schon vermutet und zu denen es Vergleichstoffe bzw. Referenzsubstanzen gibt (EP 0458622).

Verschiedene Verfahren zum Zusammenfassen von Peaks existieren (z.B. werden Schachbrettmuster über Spektrogramme gelegt), eigentlich werden diese aber vorwiegend z.B. für Genomanalysen verwendet (dabei bestehen die Datensätze aus festgelegten Genen, deren Existenz bekannt ist und lediglich das Vorhandensein beim Individuum nachzuweisen ist). Eine Clusteranalyse zum Zusammenfassen von Peaks aus Spektrogrammen wurde von V. Ruzsanyi beschrieben (Diss. 2005). Diese im Stand der Technik bestehenden Verfahren zur Analyse von VOC's in gasförmigen Proben sind kompliziert, wenig genau und haben darüber hinaus den Nachteil, dass die Probenentnahme unter absolut den gleichen Voraussetzungen durchgeführt werden muss. Vor allem bei der Diagnostik von Atemluft ist dies problematisch. Schon das Verwenden einer anderen Zahncreme bzw. das Kauen eines Kaugummis kann die Messergebnisse durcheinander bringen und zu ungenauen bzw. falschen Ergebnissen führen. Weiterhin kann Keimbefall im Mund die Ergebnisse beeinflussen und muss deshalb bei der Auswertung einer Messung auch ausgeschlossen werden, wobei die Kenntnis der beeinflussenden Moleküle wichtig ist. Daher ist eine langwierige Vorbereitung für die Probenentnahme erforderlich.

Mit diesem Stand der Technik ist es nur möglich, vordefinierte Substanzen zu bestimmen bzw. wiederzufinden. Es ist aber auch möglich, Substanzen zu finden, zuzuordnen und ihre Verhältnisse zueinander aus Spektrogrammen auszuwerten, wenn die Substanzen nicht vorbestimmt bzw. vordefiniert wurden (Purkhart et al., J. Breath Res., Bd. 5 (2011), S. 1-10). Somit ist es Aufgabe der vorliegenden Erfindung, die erhaltenen Spektrogramme von gasförmigen Proben auf das Vorliegen von unbekannten Substanzen zu analysieren, indem das Vorkommen bestimmter, gleicher Peaks bestimmten Mustern zugeordnet wird, also ein bestimmtes, gruppenspezifisches, Muster erkannt wird und entsprechende Veränderungen oder Abweichungen von Mustern und daraus folgend von physiologischen Zuständen erkannt werden.

Dabei wird von der Idee ausgegangen, dass bestimmte physiologische oder pathologische Vorgänge im Organismus, z.B. Krankheiten, Leberstoffwechsel, Nierenfunktionsstörungen, Verdauungsprozesse, Bakterienwachstum, zur Bildung und Ausscheidung bestimmter Substanzen führen, die in der Ausatemluft als charakteristische Muster von Peaks auffindbar sind. Da es sich bei diesen Peaks in den Spektren um diskrete Substanzen oder deren Metabolite handelt, wird davon ausgegangen, dass es möglich ist, Peaks anderer Herkunft oder Bedeutung davon klar abzugrenzen.

Diese Aufgabe wird anhand der unabhängigen Ansprüche 1 und 7 gelöst. Weitere vorteilhafte Ausgestaltungen, Aspekte und Details der Erfindung ergeben sich aus den abhängigen Ansprüchen, der Beschreibung, den Beispielen und den Figuren. Es wurde gefunden, dass Spektrogramme einer Probe mit diskreten Messwerten Muster ergeben, welche abgelegt in einer Datenbank und nach automatischer Kategorisierung mittels einer Auswertesoftware die Zuordnung weiterer Proben ermöglichen. Es hat sich weiterhin herausgestellt, dass die Zuordnung weiterer Proben möglich ist, ohne dass die Identifikation einzelner Substanzen erforderlich ist. Es hat sich weiterhin herausgestellt, dass die automatische Zuordnung von Spektrogrammen die Diagnose von Krankheiten unterstützen kann. Weiterhin war es von Vorteil, dass die Zuordnung von Spektrogrammen zu bestimmten Gruppen schnell erfolgen kann, wenn die Vorrichtung zur spektrometrischen Untersuchung der Probe eine Auswerteeinheit mit einer Datenbank und einer Auswertesoftware enthält.

Die Erfindung ist vorteilhaft, weil gasförmige Proben auf ihre Inhaltsstoffe analysiert werden können, ohne dass Substanzen und die dazugehörigen Peaks vorherbestimmt werden müssen. Die Erfindung ist weiterhin vorteilhaft, weil sie die Erkennung der Veränderungen von physiologischen Zuständen oder von Krankheiten durch eine Zuordnung zu gruppenspezifischen Mustern ermöglicht. Vorteilhaft ist auch, dass die Zuordnung erfolgen kann, ohne dass die Identifikation einzelner Substanzen erforderlich ist.

Ein wesentlicher Gegenstand der Erfindung ist ein Verfahren, bei dem es sich bei den zu ermittelnden Werten um diskrete Messwerte handelt und nur diese im Rahmen der Mustererkennung untersucht werden. Der Begriff des Musters bezieht sich dabei auf eine Anordnung oder Ausprägung, die sich aus dem Vorhandensein oder Fehlen von Messwerten ergibt. Mit anderen Worten bezieht sich der Begriff des Musters dabei auf Regelmäßigkeiten, die sich aus dem Vorhandensein, Fehlen und/oder aus unterschiedlichen Ausprägungen von Messwerten ergeben. Die erfassten bzw. gemessenen Werte ergeben also ein Muster, welches charakteristisch ist und wiedererkannt werden kann.

Gemäß der Erfindung werden gasförmige Proben und/oder Proben mit leicht flüchtigen Substanzen untersucht. Es kann sich dabei auch um Proben handeln, die aus dem festen oder flüssigen in den gasförmigen Aggregatzustand gebracht wurden.

Das Verfahren zur Kategorisierung von gasförmigen Proben und/oder Proben mit flüchtigen Substanzen ist dabei dadurch gekennzeichnet, dass eine Probe in einem definierten Volumen aufgenommen und chromatographisch aufgetrennt wird, von der Probe Spektrogramme erzeugt, in einer Datenbank abgelegt und mittels einer Auswertungssoftware automatisch kategorisiert ausgewertet werden, wobei die kategorisierten Spektrogramme die Zuordnung weiterer Proben ermöglichen, ohne dass die Identifikation einzelner Substanzen erforderlich ist.

Das erfindungsgemäße Verfahren besteht aus den folgenden Schritten:
1. Schritt: Automatische Erkennung von bekannten und/oder unbekannten Peaks (z.B. über lokale Maxima) in Einzelmessungen
2. Schritt: Peaks gleicher Substanzen in verschiedenen Messungen zusammenfassen/anordnen (z.B. über Clusteranalyse), um geräte- oder umweltbedingte Abweichungen auszugleichen.
3. Schritt: Peaks/Cluster mit guten Trennungseigenschaften zwischen vorgegebenen Gruppen identifizieren (z.B. Fisher-Diskriminante)
4. Schritt: den Klassifikator trainieren, welcher Muster in neuen Messungen automatisch identifizieren kann (z.B. SVM (Support Vector Machine)), um diese einer Gruppe zuzuordnen.

In dem erfindungsgemäßen Verfahren zur automatischen Klassifizierung (bzw. Kategorisierung) von gasförmigen Proben werden diese in einem ersten Schritt zunächst mit spektrometrischen Verfahren analysiert. In einem zweiten Schritt werden in den gemessenen Spektrogrammen automatisch relevante Muster für verschiedene vorgegebene (zu überprüfende) Klassen gesucht. Wesentlich im Sinne der Erfindung ist, dass die Identifizierung einzelner Substanzen nicht mehr nötig bzw. unwichtig ist. Erfindungsgemäß werden die zu analysierenden flüchtigen organischen Verbindungen in der Probe semiquantitativ bestimmt. Unter einem definierten Volumen wird ein Volumen verstanden, das vom Anwender vorgegeben ist. Mit anderen. Worten wird für eine oder mehrere Messungen ein Volumen am Gerät eingestellt oder anders vorgegeben, welches dann als definiert für diese eine oder mehrere Messungen bezeichnet wird. Die Probe wird also vor der Messung quantifiziert. Dies ist dadurch bedingt, dass die Messempfindlichkeit eines beliebigen Messgerätes durch die pro Einzelmessung angebotene Menge des bzw. der gesuchten Analyten bestimmt wird. In Proben von Ausatemluft sind diese Analyten in einer bestimmten Luftmenge enthalten. Die Konzentration des Analyten in der Ausatemluft hängt von de Menge der Absonderung des Stoffes in den Atemwegen und der Lunge pro Zeiteinheit des in dieser Zeiteinheit geatmeten Luftvolumens ab, das diesen Stoff aufnimmt und transportiert. Im Messgerät bzw. der immer vorgeschalteten Probenaufnahmebehälter (d.h. Probenkammer, Trap, Probeschleife, Extraktionssäule) wird der Analyt aus der Luft an der Oberfläche des Füllmaterials der Trap temporär festgehalten. Es ist leicht einsehbar, dass sich bei einer Verdopplung der Luftmenge die Menge des verfügbaren Analyten auch verdoppelt, oder bei einer Halbierung der Luftmenge entsprechend halbiert. Mit anderen Worten kann eine quantitative Bestimmung eines Analyten nur gelingen, wenn die applizierte Probenmenge bekannt ist (definiertes Volumen), d.h. eine quantitative Bewertung gemessener Peaks ist also nur vergleichbar zwischen zwei Messungen wenn die gleiche Probenmenge bei der gleichen Geräteeinstellung des Messgeräts verwendet wurde. Die Bestimmung eines Analyten erfolgt dabei semiquantitativ, indem die die Peaks im Verhältnis zu anderen Peaks erfasst werden. Dabei muss sich die Stärke eines Peaks nicht auf ein bestimmtes Volumen beziehen.

Die Spektrogramme werden mittels herkömmmlicher Methoden, also lonenbeweglichkeitsspektrometrie (IMS), differentielle lonen-Mobilitäts-Spektrometrie (DMS), Gaschromatographie (GC) erzeugt. Gemäß der Erfindung kann die gasförmige Probe eine beliebige Probe sein. Dabei ist es bevorzugt, wenn die gasförmige Probe aus der Umgebungsluft entnommen wird. Es ist noch mehr bevorzugt, wenn die gasförmige Probe dem Abgas einer technischen Anlage entnommen wurde. Es ist noch mehr bevorzugt, wenn die gasförmige Probe Ausatemgas ist, und ganz bevorzugt ist es, wenn die gasförmige Probe menschliches Ausatemgas ist. Als Ausatemgas wird dabei das Gas bezeichnet, das bei der Atmung die Lunge verlässt.

Zur Auswertung wird eine automatische Erkennung der Peaks in Einzelmessungen durchgeführt. Daraufhin erfolgt eine Zusammenfassung, von Peaks gleicher Substanzen in verschiedenen Messungen und eine Identifizierung von Peaks/ Clustern mit Trennungsmöglichkeiten zwischen vorgegebenen Gruppen. Weiter werden eine Merkmalsreduktion und eine Klassifizierung durchgeführt, wobei der Klassifikator trainiert wird um Muster in neuen Messungen automatisch zu klassifizieren und Gruppen zuzuordnen.

Mit anderen Worten wird bei dem erfindungsgemäßen Verfahren auf der Basis der in der Datenbank abgelegten und kategorisierten Spektrogramme eine automatische Zuordnung der Peaks der Spektrogramme in Einzelmessungen sowie eine Zuordnung von Peaks in verschiedenen Messungen erfolgen, wobei eine Identifizierung von Mustern mit automatischer Zuordnung zu bereits kategorisierten Gruppen sowie eine'Erfassung neuer Muster erfolgen, wobei unspezifische Signale automatisch ausgeblendet werden, und wobei der Klassifikator trainiert wird um Muster in neuen Messungen automatisch zu klassifizieren und Gruppen zuzuordnen. Dabei werden mittels der Auswertungssoftware Daten aus Ionenbeweglichkeitsspektrometrie und Gaschromatographie miteinander in Verbindung gesetzt und auf Gemeinsamkeiten oder Unterschiede, wie Verteilung der Werte, Mustererkennung und / oder Erkennung von zusammengehörigen Peaks untersucht. Vorzugsweise wird die Ausprägung bestimmter bekannter Datenpunkte bezüglich Höhe des Messwertes und/ oder Häufigkeit des Auftretens untersucht. Dabei werden die Daten verglichen, Merkmale erkannt, markiert und / oder ausgeschlossen und unbekannte Einzeldatenfiles einer schon bekannten Gruppe gleicher Merkmalsausprägung zugeordnet.

In einer weiteren Ausführungsform des erfindungsgemäßen Verfahrens werden mittels einer Datenbank Positionen von Peaks bestimmter Substanzen gespeichert, um diese in unbekannten Messungen wiederzufinden.

Erfindungsgemäß wird das Verfahren zur Bestimmung von Substanzen in gasförmigen Proben eingesetzt. Bevorzugt findet es seine Anwendung zur Bestimmung von physiologischen Zuständen oder zur Diagnose von Krankheiten bei Menschen oder Tieren.

Ferner betrifft die Erfindung auch eine Vorrichtung zur Durchführung des erfindungsgemäßen Verfahrens, welche ein Behältnis zum Ansaugen und Sammeln der Probe, eine Vorrichtung zur Durchführung der chromatographischen Auftrennung, ein Spektrometer, sowie eine Auswerteeinheit mit einer Auswertungssoftware enthält.

In einer bevorzugten Ausführungsvariante der Erfindung kann das Behältnis zum Ansaugen und Sammeln der Probe und zu deren Aufbewahrung in einem Probensammelgefäss räumlich getrennt von der Analyse angebracht werden. Die Analyse kann dann auch zeitlich getrennt vom Ansaugen und Sammeln der Probe vorgenommen werden. Beispielsweise kann die Probe dabei im Gefäss zum Analysegerät transportiert werden. Dort erst wird die Probe aus dem Behältnis in die Analyseeinheit gegeben und aufgetrennt.

Weiterhin bevorzugt ist eine Ausführungsvariante, in der die Vorrichtung einen Behälter zum Ansaugen und Sammeln der Probe und zu deren Aufbewahrung in einem Probensammelgefäß umfasst, so dass die Probennahme räumlich und zeitlich getrennt von der Analyse erfolgen kann, und weiterhin eine Vorrichtung zur Durchführung der chromatographischen Auftrennung, ein Spektrometer, sowie eine Auswerteeinheit mit einer Datenbank und einer Auswertungssoftware zur Zuordnung der Proben zu einem bestimmen Muster enthält.

In einer weiteren bevorzugten Ausführungsform ist das Behältnis zum Ansaugen und Sammeln der Probe und zu deren zeitlich befristeter Aufbewahrung ein Probensammelgefäß, welches über mindestens zwei getrennte Öffnungen verfügt, wobei eine erste Öffnung zum Probeansaugen und eine zweite Öffnung zum Absaugen dienen. Diese Ausführungsform ist deshalb vorteilhaft, weil die Probenentnahme räumlich und zeitlich getrennt von der Analyse ermöglicht wird. Dadurch ist gewährleistet, dass ein Patient, dessen Ausatemgas oder andere Probe genommen wird, keinen Kontakt zum Gerät hat. Auf diese Weise können die Kosten für die Medizinproduktezulassung niedrig gehalten werden.

In einer weiteren bevorzugten Ausführungsform ist die Vorrichtung dadurch gekennzeichnet, dass Messergebnisse, die mit verschiedenen Geräten oder verschiedenen Geräteeinstellungen gewonnen wurden, nach einem bestimmten Verfahren miteinander vergleichbar sind und damit speziell bei Screeninguntersuchungen oder Verlaufskontrollen als geräteunabhängig festgelegte Muster (Krankheitsmuster oder Muster verschiedener VOC) dokumentierbar sind. Die Abkürzung VOC ist dabei ein Fachbegriff und steht für flüchtige organische Verbindungen (engl. volatile organic compounds), also für organische Stoffe, die leicht verdampfen bzw. schon bei niedrigen Temperaturen (z.B. Raumtemperatur) als Gas vorliegen.

Dabei ist es denkbar, dass das beschriebene Auswerteverfahren mit einer standardisierten Probenentnahme verbunden wird, da die Vergleichbarkeit der Messungen, z.B. der Ausatemluft, essentiell von der Probenentnahme abhängig ist.

In einer alternativen ebenfalls bevorzugten Variante können die Ergebnisse von Einzelproben verschiedener Herkunft, welche aber mit den eigens verwendeten Messverfahren hergestellt sind, den schon vorhandenen Vergleichsmessungen zugeordnet werden (Klassifikation). Bei dem Vorhandensein von Vergleichsmessungen (Lernstichprobe der gesuchten VOC-Muster) ist in neuen Messungen klassifizierbar, mit welcher Sicherheit die Merkmale der Vergleichsstichprobe vorhanden sind. Mit anderen Worten ist bevorzugt, wenn Messergebnisse von Einzelproben verschiedener Herkunft den schon vorhandenen Vergleichsmessungen (Lernstichprobe der gesuchten VOC-Muster) zuordenbar sind (Klassifikation) und bei Vorhandensein von Vergleichsmessungen für neue Messungen berechenbar/abschätzbar ist, mit welcher Sicherheit die Merkmale der Vergleichsstichprobe vorhanden sind.

In einer bevorzugten Anwendungsform des erfindungsgemäßen Verfahrens kann beispielsweise die Zuordnung einer Probe zu Diabetes, bestimmten bakteriellen Infektionen, Tumorerkrankungen, Stoffwechselstörungen, Leberfunktionsstörung Nierenfunktionsstörung (Niereninsuffizienz) und /oder der Einnahme von Medikamenten o.ä. zugeordnet werden.

Die Erfindung verwendet eine universelle Auswertesoftware für das Einlesen, die Datenreduktion und die Auswertung von multiplen Spektren oder Datenfiles in spezifischer Anordnung der Daten, die analog zu Spektren angeordnet sind. Dabei handelt es sich um unbekannte Daten, die während der Auswertung nach gemeinsamen Mustern untersucht und geordnet werden können.

Die angebotenen Daten können dabei auch aus verschiedenen Quellen stammen. Die Werte werden automatisch auf ein gemeinsames vergleichbares Niveau transformiert, z.B. durch Normierung auf bekannte Einzelwerte oder Normierung auf eine sog. RIP (reactant ions peak, dt. Reaktantionenpeak) der in allen Messungen oder Datenfiles vorhanden ist.

Die unterschiedlichen Daten, sind Spektren aus lonenbeweglichkeitsspektrometrie und Gaschromatographie. Weiter ist es mit der Auswertesoftware möglich, die mit verschiedenen adaptierten Standardmethoden mehrdimensional geordnete Daten auf Gemeinsamkeiten oder Unterschiede in Datenstruktur (Verteilung der Werte, Mustererkennung, Erkennung von zusammengehörigen Peaks), Ausprägung bestimmter bekannter Datenpunkte (Peaks) bezüglich Höhe des Messwertes oder Häufigkeit des Auftretens zu untersuchen.

Dabei ist es möglich beim Vergleich großer geordneter Datenmengen gemeinsame Merkmale zu erkennen und. zu markieren, sowie unwichtige oder nur zufällig auftretende Merkmale auszuschließen. Mittels der Software können fernerhin unbekannte Einzeldatenfiles einer schon bekannten Gruppe gleicher Merkmalsausprägung zugeordnet werden. Umwelteinflüsse werden dabei automatisch ausgeblendet, so weit sie sich nicht mit den Probenpeaks überlagern.

Bekannte Messungen (z.B. von Studien) werden dabei in einer Datenbank organisiert, um schnell auf sie zugreifen zu können und gegebenenfalls neue Muster zu berechnen. Bei einer ausreichend großen Datenbank sind auch Verlaufsanalysen der Zustände eines Patienten oder auch einer technischen Anlage möglich. Prinzipiell ist die Datenbank aus vorher analysierten Proben, besonders Ausatemproben, aufbaubar.

Das Verfahren dient also besonders und bevorzugt zur Erkennung von Mustern von Biomarkern oder Markern im weiteren Sinne in der Ausatemluft von Lebewesen. Hierzu wird zunächst ein definiertes Volumen des Atems gesammelt und mit einem spektrometrischen Verfahren analysiert. Das gewonnene Spektrogramm wird danach automatische mit Hilfe einer Biomarkerdatenbank ausgewertet und kategorisiert.

So ist es durch das erfindungsgemäße Verfahren sowie die erfindungsgemäße Vorrichtung erstmals möglich geworden, ohne langwierige Probenvorbereitung die flüchtigen Inhaltsstoffe einer gasförmigen Probe zu bestimmen. Es ist damit möglich, ein Routineverfähren für jeden Tag und sämtliche Lebensumstände bereitzustellen, welches sicher und zuverlässig funktioniert.

Die Erfindung wird nachfolgend anhand der Ausführungsbeispiele und der Figuren näher erläutert, wobei der Gegenstand der Erfindung nicht auf die Ausführungsbeispiele und die Figuren beschränkt ist. Es stellen dar:

### Ausführungsbeispie) 1

### Vergleich GC-DMS vs. DMS vs. IMS

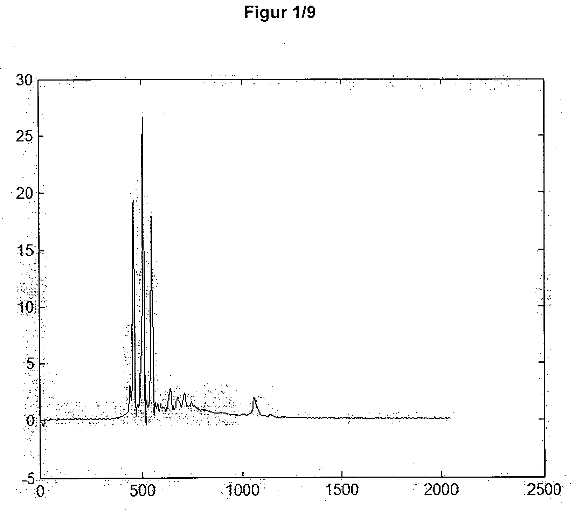

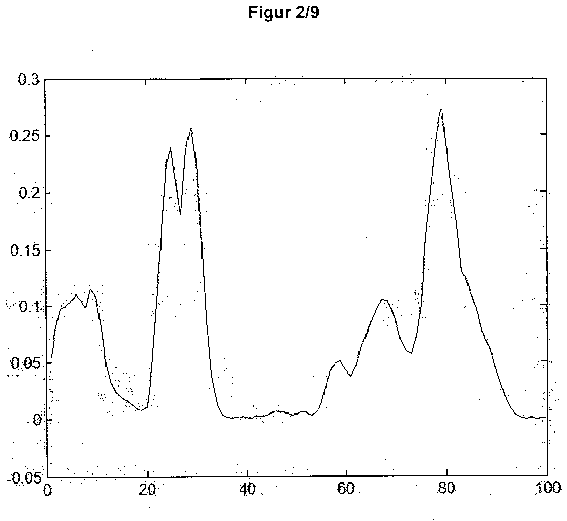

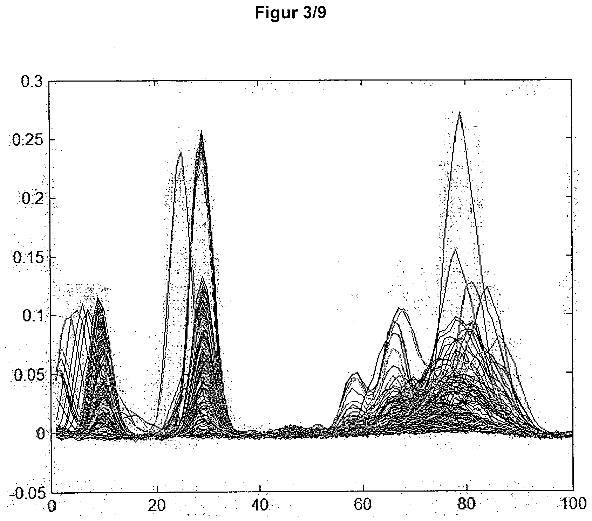

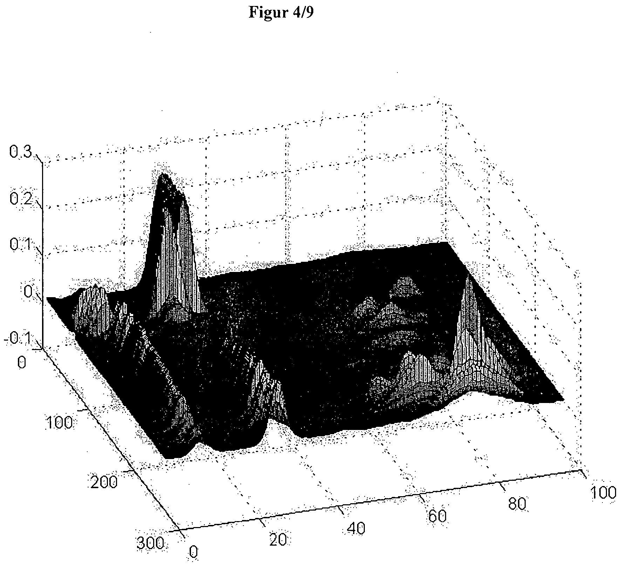

Beispiel für mögliche Datentransformierung von einem Messgerät für ein anders zum Vergleich der Messungen:
IMS - Messung Ziegenkot (einige Messungen gemittelt; positiv):
   (Ionenbeweglichkeitspektrometrie, nur positiv, keine Vorsäule) siehe Figur 1.
DMS - Messung Ziegenkot (gefiltert, da GC-Trennung nachträglich wieder zusammengefasst; positiv)
   (DMS, alle Messungen sind zu einem Spektrum zusammengefasst, was etwa der Situation ohne Vorsäule entspricht) **siehe** Figur 2.
GC-DMS - Messung Ziegenkot (positiv; selbe Messung wie oben)
   Hier sind alle 270 Einzelmessungen nach Trennung über eine Vorsäule in eine Graphik aufeinander dargestellt. Aus diesem Bild wurde das Bild auf der Seite zuvor (s.o.) erstellt. **Siehe** **Figuren 3** **und** **4****.**

### Ausführungsbeispiel 2

### DMS Messung an gesunden Probanden:

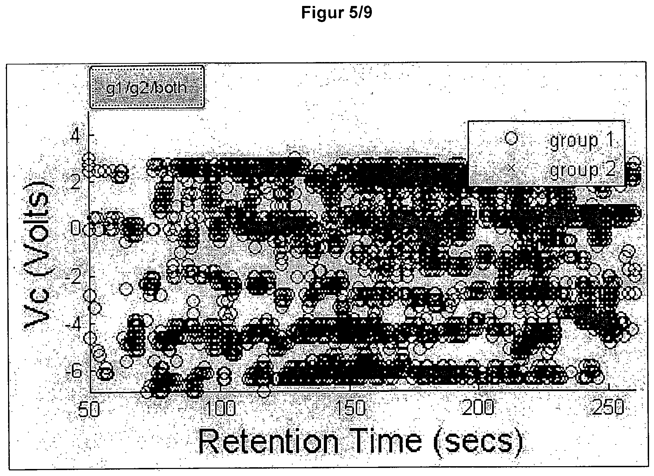

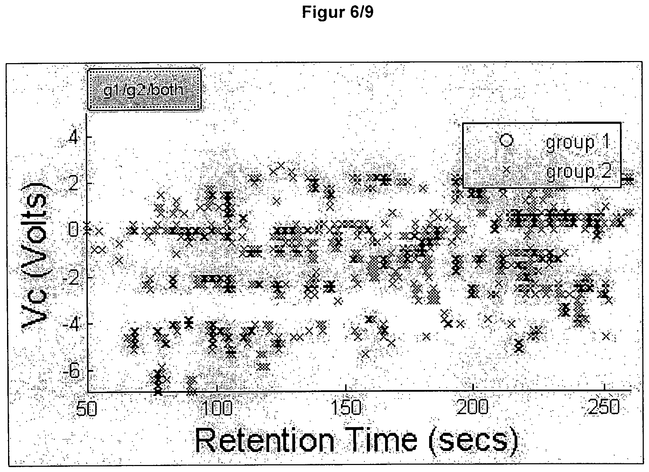

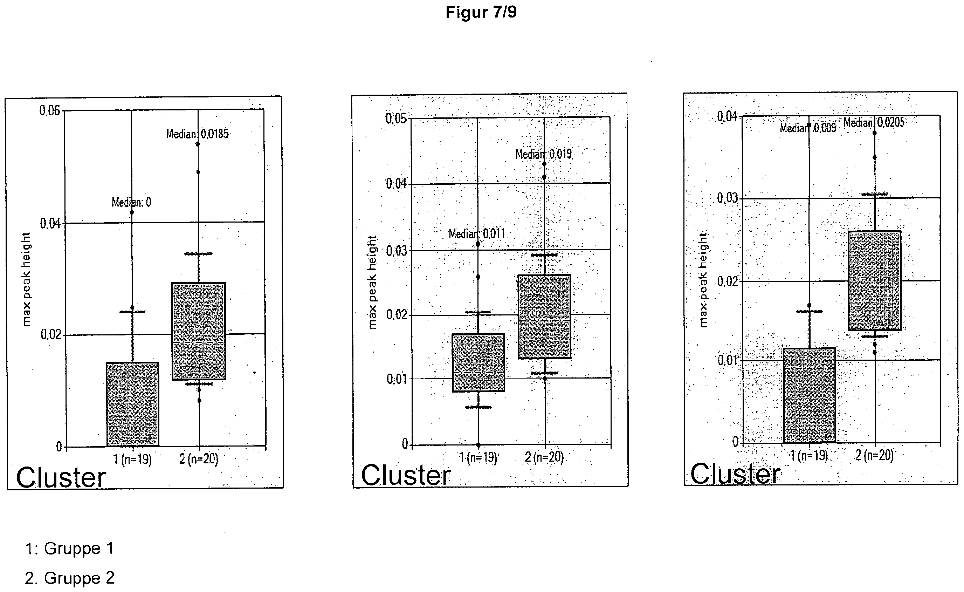

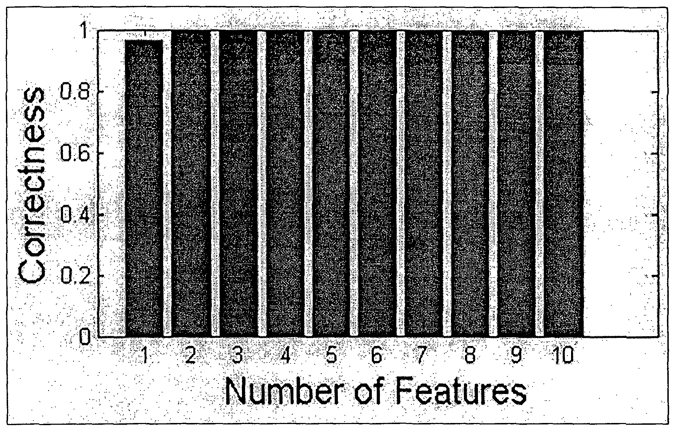

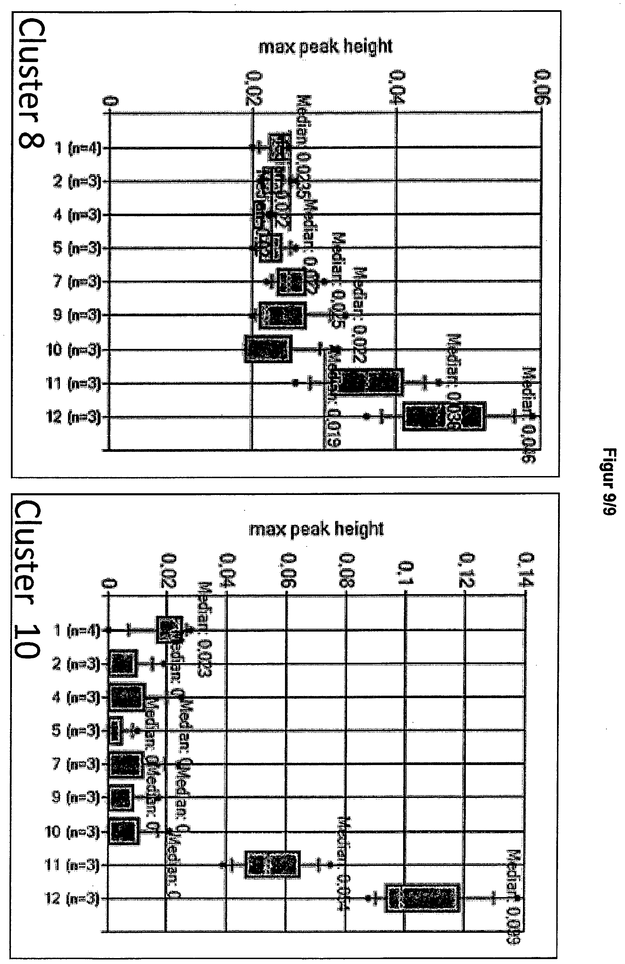

Alle Messungen an einer bestimmten Zahl Probanden werden in zwei Abbildungen (Figur 5 und 6) zusammengenommen (Darstellung aller (!) Peaks aller Probanden jeder Gruppe in je einer Abbildung).
Diese Peaks werden dann in Cluster zusammengefasst (Figur 7), wobei davon ausgegangen wird, dass jedes Cluster gleiche chemische Marker bzw. gleiche Merkmale jedes Probanden zusammenfasst.
Danach kann dann für jedes Cluster, bestehend aus Peaks mit unterschiedlichen Werten (Peakhöhe) der Unterschied zwischen den Gruppen berechnet und dargestellt werden (Boxplot) (Figur 8, 9).

Weiter kann dann an Hand der Ergebnisse dieser Berechnung eine Wichtung der Cluster für die Trennung zwischen den Gruppen vorgenommen werden.

Wenn diese Daten vorliegen, können dann unbekannte neue Einzelmessungen daraufhin untersucht werden, zu welcher bekannten Gruppe diese Einzelmessung am ehesten zugeordnet werden kann.

### Abbildungsverzeichnis

Figur 1/9: **IMS - Messung Ziegenkot.** Ohne Vorsäule, einige Messungen gemittelt
Figur 2/9: **DMS - Messung Ziegenkot.** Alle Messungen sind zu einem Spektrum zusammengefasst.
Figur 3/9: **GC-DMS - Messung Ziegenkot.** 2D-Darstellung der 270 Einzelmessungen nach Trennung über eine Vorsäule.
Figur 4/9: **GC-DMS - Messung Ziegenkot.** 3D-Darstellung der 270 Einzelmessungen nach Trennung über eine Vorsäule.
Figur 5/9: **DMS-Messung von gesunden Probanden (Gruppe 1).** Analyse der in der ausgestoßenen Atemluft enthaltenen flüchtigen organischen Verbindungen (Vc). Alle Peaks einer Gruppe (Gruppe 1) zusammengefasst aus allen Einzelmessungen.
Figur 6/9: **DMS-Messung von gesunden Probanden (Gruppe 2).** Analyse der in der ausgestoßenen Atemluft enthaltenen flüchtigen organischen Verbindungen (Vc).. Alle Peaks einer Gruppe (Gruppe 2) zusammengefasst aus allen Einzelmessungen.
Figur 7/9: **Zusammenfassung der Peaks zu Clustern.** Trennende Cluster der beiden Gruppen im Vergleich, wobei davon ausgegangen wird, dass jedes Cluster gleiche chemische Marker bzw. gleiche Merkmale jedes Probanden zusammenfasst.
Figur 8/9: **Clusteranalyse der Gruppen 1 und 2.** Unterschiede zwischen Klassifikation der beiden Gruppen anhand der x-best-trennenden Cluster (Feature) ermittelt durch Kreuzvalidierung.
Figur 9/9: **Clusteranalyse der Gruppen 1 und 2.** Ausgewählte Cluster (z.B. 8 und 10) zeigen Unterschiede in den Peakhöhen bei verschiedenen Probanden.

## Patentansprüche

1. Verfahren zur Kategorisierung von gasförmigen Proben, **dadurch gekennzeichnet, dass** eine Probe in einem definierten Volumen aufgenommen und chromatographisch aufgetrennt wird, von der Probe Spektrogramme erzeugt, in einer Datenbank abgelegt und mittels einer Auswertungssoftware automatisch kategorisiert werden, wobei die kategorisierten Spektrogramme für die Zuordnung weiterer Proben verwendet werden, ohne die Identifikation einzelner Substanzen und wobei die Spektrogramme mittels Gaschromatographie in Verbindung mit lonenbeweglichkeitsspektrometrie (IMS) oder Differentieller lonenbeweglichkeitsspektrometrie (DMS) erzeugt werden, und mittels der Auswertungssoftware:
- auf Gemeinsamkeiten oder Unterschiede, nämlich Verteilung der Werte. Mustererkennuna, Erkennung von zusammengehörigen Peaks,-analysiert werden und die Substanzen semi^{g}uantitativ bestimmt werden, indem die Verhältnisse der Peaks zu anderen Peaks erfasst werden
- die Ausprägung bestimmter bekannter Datenpunkte bezüglich Höhe des Messwertes und / oder Häufigkeit des Auftretens untersucht werden und
- die Daten verglichen, Merkmale erkannt, markiert und / oder ausgeschlossen werden und
- unbekannte Einzeldatenfiles einer schon bekannten Gruppe gleicher Merkmalsausprägung zugeordnet werden und
- auf der Basis, der in der Datenbank abgelegten und kategorisierten Spektrogrammen eine Identifizierung von Mustern mit automatischer Zuordnung zu bereits kategorisierten Gruppen sowie eine Erfassuna neuer Muster erfolgen, wobei unspezifische Signale, hervorgerufen durch Umwelteinflüsse, automatisch ausgeblendet werden, und wobei der Klassifikator trainiert wird um Muster in neuen Messungen automatisch zu klassifizieren und Gruppen zuzuordnen.

2. Verfahren nach dem vorangegangenen Anspruch wobei die gasförmige Probe der Umgebungsluft entnommen wird.

3. Verfahren nach einem der vorangegangenen Ansprüche, wobei die gasförmige Probe Abgas oder Abluft einer technischen Anlage ist.

4. Verfahren nach einem der vorangegangenen Ansprüche, wobei die gasförmige Probe Ausatemgas ist.

5. Verfahren Anspruch 4, wobei die gasförmige Probe menschliches Ausatemgas ist.

6. Anwendung des Verfahrens nach einem der vorangegangene Ansprüche zur Unterstützung der Bestimmung von physiologischen Zuständen oder der Diagnose von Krankheiten bei Menschen oder Tieren.

7. Vorrichtung eingerichtet zur Durchführung des Verfahrens nach einem der Ansprüche 1 - 6, **dadurch gekennzeichnet, dass** sie einen Behälter zum Ansaugen und Sammeln der Probe und zu deren Aufbewahrung in einem Probensammelgefäß umfasst, so dass die Probennahme räumlich und zeitlich getrennt von der Analyse erfolgen kann, und weiterhin eine Vorrichtung zur Durchführung der chromatographischen Auftrennung, ein Spektrometer, sowie eine Auswerteeinheit mit einer Datenbank und einer Auswertungssoftware zur Zuordnung der Proben zu einem bestimmen Muster enthält.

8. Vorrichtung nach Anspruch 7 **dadurch gekennzeichnet, dass** das Behältnis zum Ansaugen und Sammeln der Probe und zu deren zeitlich befristeter Aufbewahrung ein Probensammelgefäß ist, welches über mindestens zwei getrennte Öffnungen verfügt, wobei eine erste Öffnung zum Probeansaugen und eine zweite Öffnung zum Absaugen dient.

9. Vorrichtung nach einem der Ansprüche 7 oder 8, **dadurch gekennzeichnet, dass** Messergebnisse, die mit verschiedenen Geräten oder verschiedenen Geräteeinstellungen gewonnen wurden, nach einem bestimmten Verfahren miteinander vergleichbar sind, und damit speziell bei Screeninguntersuchungen oder Verlaufskontrollen geräteunabhängig festgelegte Muster (Krankheitsmuster oder Muster verschiedener VOC) dokumentierbar sind.

10. Vorrichtung nach einem der Ansprüche 7-9, **dadurch gekennzeichnet, dass** die Ergebnisse von Einzelproben verschiedener Herkunft, aber mit den eigens verwendeten Messverfahren hergestellt, den schon vorhandenen Vergleichsmessungen zuordenbar sind (Klassifikation) und bei Vorhandensein von Vergleichsmessungen (Lernstichprobe der gesuchten VOC-Muster) in neuen Messungen klassifizierbar ist, mit welcher Sicherheit die Merkmale der Vergleichsstichprobe vorhanden sind.

## Claims

1. Method for categorizing of gaseous samples, **characterized in that** a sample is taken in a defined volume and separated chromatographically, wherein spectrograms are generated, stored in a database, are automatically categorized by means of an evaluation software, wherein the categorized spectrograms are used for the assignment of further samples, without the identification of individual substances, and wherein the spectrograms are generated by gas chromatography, in conjunction with lon-mobility spectrometry (IMS) or differential Ion-mobility spectrometry (DMS), and by means of the evaluation software
- analysed for similarities or differences, namely distribution of values, pattern recognition, detection of related peaks and the substances are determined semiquantitative calculating the ratio of the peaks to other peaks.
- investigation of the expression of certain known data points regarding to the amount of the measured value and/or the frequency of their occurrence and
- the data are compared, characteristics are identified, marked and/or excluded and
- unknown individual data files are assigned to an already known group of the same characteristic expression of features and
- based on categorized spectrograms already stored in the database, will be performed the identification of patterns with automatic mapping to already categorized groups, as well as the recognition of new patterns, wherein nonspecific signals, caused by environmental influences, will be auto-hidden, and wherein the classifier is trained to classify pattern in new measurements automatically and associate to groups.

2. Proceedings of the preceding claim, wherein the gaseous sample is taken of the ambient air.

3. Proceedings under one of the preceding claims wherein the gaseous sample is exhaust or exhaust air of technical facility.

4. Proceedings under one of the preceding claims wherein the gaseous sample is exhaled air.

5. Process according to claim 4 wherein the gaseous sample is human exhaled air.

6. Application of the procedure under one of the preceding claims to support the determination of physiological conditions or diagnosis of diseases in humans or animals.

7. Device, set up to carry out the procedure in accordance with one of the preceding claims 1-6, characterized that it includes a container to suction and collect the sample and to store the sample in a reservoir, so sampling spatially and temporally may be separated from the analysis, additionally the device includes a set up to carry out the chromatographic separation, a spectrometer, as well as a unit with a database and a software to assign the samples to a specific pattern.

8. Device according to claim 7, **characterised by** the fact that the container to suction and collection of the sample and for their temporary storage is a sample reservoir which has at least two separate openings, with a first opening for the suction of the sample and a second opening which is used for vacuuming.

9. Device according to one of the claims 7 or 8, characterised that measurements which were obtained with different devices or different device settings will be comparable according to a specific procedure, and can be documented by this way specifically for screening or follow-up as a device-independent template (disease patterns or patterns of different VOC).

10. Device according to the claims 7 - 9, **characterised by** the fact that measurements of single samples of various origins, but created with specially-used measuring methods, can be classified to the already existing comparison measurements (classification) and in case of pre-existing comparison measurements (training samples of selected VOC patterns) newly taken measurements are relatable and it is classifiable with which security the same characteristics like in the comparison samples are present.

## Revendications

1. Procédé pour la catégorisation d'échantillons gazeux, **caractérisé par le fait qu'**un échantillon est incorporé dans un volume défini et séparé par chromatographie, que des spectrogrammes sont générés à partir de l'échantillon, stockés dans une base de données, catégorisés automatiquement au moyen d'un logiciel d'évaluation, les spectrogrammes catégorisés étant utilisés pour l'affectation d'autres échantillons, sans identification de chaque substance et les spectrogrammes étant générés au moyen de la chromatographie gazeuse, en relation avec la spectrométrie par mobilité ionique (IMS) ou la spectrométrie par mobilité ionique différentielle (DMS), et qu'au moyen du logiciel d'évaluation,
- une analyse des points communs ou des différences, notamment de la répartition des valeurs, de la reconnaissance de modèles, de l'identification de pics apparentés est réalisée et les substances sont déterminées semi-quantitativement par l'enregistrement des relations des pics par rapport à d'autres pics
- la caractéristique de points de données définis connus concernant la valeur de mesure et/ou la fréquence d'apparition est analysée et
- les données sont comparées, les caractéristiques identifiées, marquées et/ou exclues et
- les fichiers de données individuels inconnus d'un groupe déjà connu sont affectés à des valeurs caractéristiques identiques et
- sur la base des spectrogrammes stockés et catégorisés dans la base de données, une identification des modèles avec affectation automatique à des groupes déjà catégorisés et un enregistrement de nouveaux modèles sont réalisés, les signaux non spécifiques, causés par des influences environnementales, étant masqués automatiquement et le classificateur étant entraîné pour classifier automatiquement les échantillons dans de nouvelles mesures et les affecter à des groupes.

2. Procédé conformément à la revendication précédente, l'échantillon gazeux étant prélevé de l'air ambiant.

3. Procédé conformément à l'une des revendications précédentes, l'échantillon gazeux étant du gaz ou de l'air évacué d'une installation technique.

4. Procédé conformément à l'une des revendications précédentes, l'échantillon gazeux étant du gaz expiré.

5. Procédé conformément à la revendication n°4, l'échantillon gazeux étant du gaz expiré humain.

6. Application du procédé conformément à l'une des revendications précédentes, visant à faciliter la détermination d'états physiologiques ou le diagnostic de maladies chez l'homme ou l'animal.

7. Dispositif installé pour réaliser le procédé conformément à l'une des revendications n°1 à n°6, **caractérisé par le fait qu'**il comprend un conteneur pour aspirer et collecter l'échantillon et pour le conserver dans un récepteur d'échantillon de sorte que la prise d'échantillon puisse s'effectuer séparément de l'analyse, dans l'espace et dans le temps, et qu'il contient, par ailleurs, un dispositif pour réaliser la séparation chromatique, un spectromètre ainsi qu'une unité d'évaluation avec une base de données et un logiciel d'évaluation pour l'affectation des échantillons à un modèle défini.

8. Dispositif conformément à la revendication n°7, **caractérisé par le fait que** le conteneur pour aspirer et collecter l'échantillon et pour le conserver temporairement est un récepteur d'échantillon qui dispose au moins de deux ouvertures séparées, une première ouverture servant à l'absorption de l'échantillon et une deuxième ouverture à l'aspiration.

9. Dispositif conformément à l'une des revendications n°7 ou n°8, **caractérisé par le fait que** les résultats de mesure qui ont été obtenus avec des appareils ou des réglages d'appareils différents, sont comparables selon un procédé défini et peuvent être, par conséquent, documentés, notamment pour les examens de dépistage ou les contrôles d'évolution des modèles définis indépendamment des appareils (modèle de maladie ou modèle de divers COV).

10. Dispositif conformément à l'une des revendications n°7 ou n°9, **caractérisé par le fait que** les résultats de mesure d'échantillons isolés d'origines différentes, mais obtenus à l'aide de procédés de mesure spécialement utilisés, sont affectables aux mesures de comparaison déjà existantes (classification) et, en cas de présence de mesures de comparaison (échantillon d'apprentissage des modèles de COV recherchés), sont classifiables dans de nouvelles mesures, avec la sécurité des caractéristiques de l'échantillon de comparaison qui sont disponibles.
